(19) Europäisches Patentamt
European Patent Office
Office européen des brevets

(11) Veröffentlichungsnummer: **0 272 734**
**A2**

(12) **EUROPÄISCHE PATENTANMELDUNG**

(21) Anmeldenummer: **87202412.0**

(22) Anmeldetag: **07.06.85**

(51) Int. Cl.4: **C07D 307/30**, C07D 307/32, C07H 15/252, A61K 31/70

(30) Priorität: **15.06.84 FR 8409405**

(43) Veröffentlichungstag der Anmeldung: **29.06.88 Patentblatt 88/26**

(60) Veröffentlichungsnummer der früheren Anmeldung nach Art. 76 EPÜ: **0 170 820**

(84) Benannte Vertragsstaaten: **AT BE CH DE GB IT LI LU NL SE**

(71) Anmelder: **LABORATOIRES HOECHST S.A.**
**1 Terrasse Bellini**
**F-92800 Puteaux(FR)**

(72) Erfinder: **Florent, Jean-Claude**
**23 rue des Causses**
**F-91940 Les Ulis(FR)**
Erfinder: **Gesson, Jean-Pierre**
**La Germonière Montamisé**
**F-86360 Chasseneuil-du-Poitou(FR)**
Erfinder: **Jacquesy, Jean-Claude**
**46 rue du Planty**
**F-86180 Buxerolles(FR)**
Erfinder: **Mondon, Martine**
**9 rue du Pontreau**
**F-86000 Poitiers(FR)**
Erfinder: **Monneret, Claude**
**9 Avenue Lamorcière**
**F-75012 Paris(FR)**

(74) Vertreter: **Orès, Bernard et al**
**Cabinet ORES 6, Avenue de Messine**
**F-75008 Paris(FR)**

(54) **Neue 3-amino-3,4,6-tridesoxyglykale, Verfahren zu ihrer Herstellung und mittels dieser Glykale erhältliche Anthracycline.**

(57) Verfahren zur Herstellung neuer 3-Amino-3,4,6-tridesoxyglykale und Verwendung dieser neuen Verbindungen zur Herstellung von Anthracyclinen.

Die neuen Glykale gemäss den untenstehenden allgemeinen Formeln 14 und 15 werden durch Reaktion der Glykale der Formeln 10 und 11 mit einem Gemisch aus Natriumhydrid und Schwefelkohlenstoff und nachfolgender Reaktion mit Methyljodid unter Gewinnung von Methylthiocarbonaten der allgemeinen Formeln 12 und 13 hergestellt, worin die Methylthiocarbonatgruppen durch Reduktion mittels Tributylzinnhydrid in Gegenwart von Azobisisobutyronitril eliminiert werden.

Anwendungsbeispiele: Verwendung von Glykalen der Formeln 14 und 15 zur Herstellung von Anthracyclinen; Verwendung der letztgenannten Verbindungen zur Herstellung von Arzneimitteln.

EP 0 272 734 A2

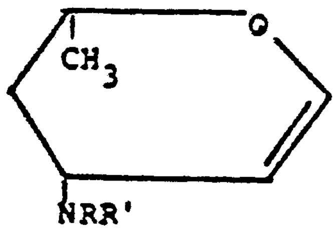

14

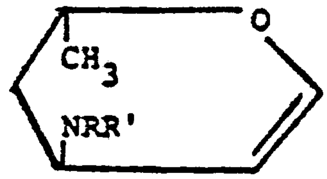

15

# Neue 3-Amion-3,4,6-tridesoxyglykale, Verfahren zu ihrer Herstellung und mittels dieser Glykale erhältliche Anthracycline

Gegenstand der vorliegenden Erfindung sind neue 3-Amino-3,4,6-tridesoxyglykale, Verfahren zu ihrer Herstellung sowie mittels dieser Glykale erhältliche Anthracycline.

Die Anthracycline stellen eine sehr bedeutende Gruppe von antibiotischen Arzneimitteln und Präparaten zur Krebsbekämpfung dar. Zu dieser Gruppe gehören beispielsweise Doxorubicin als antibiotisches Zytostatikum hoher Wirksamkeit, sowie das Daunorubicin, welches als antibiotisches, antimitotisches Mittel insbesondere gegen Leukämien, Morbus Hodgkin, Retikulumsarkom oder Lymphosarkome gilt.

Es sind schon zahlreiche Forschungen über diese Stoffe durchgeführt worden, um ihre Struktur-und biologische Wirkungsbeziehungen aufzuklären. Diese Forschungen machen deutlich, dass die Abwandlung von Zuckerderivaten häufig zu analog strukturierten Verbindungen führt, welche pharmakologisch noch interessanter sind als die Ausgangsverbindungen. Diese Abwandlungen bewirken eine Herabsetzung der therapeutischen Wirkdosis, eine ausgeprägtere Antitumorwirksamkeit, sowie eine Verringerung der Nebenwirkungen, insbesondere der kardiotoxischen Begleitwirkung (vergl. insbes. die Arbeiten von F. Arcamone "Doxorubicin", Academic Press, New York, 1981, über Daunosamin, die Zucker von Daunorubicin und Doxorubicin oder Adriamycin).

Unter den bereits vorgeschlagenen Abwandlungen spielt u.a. die Desoxydierung des in 4'-Stellung befindlichen Kohlenstoffatoms das Zuckers (vergl. EP-B-48 967 von Farmitalia) eine Rolle; nach Aussage der Verfasser dieses Patents zeigt das durch die untenstehende Formel I wiedergegebene Produkt 3 eine ausserordentlich interessante Antitumorwirksamkeit.

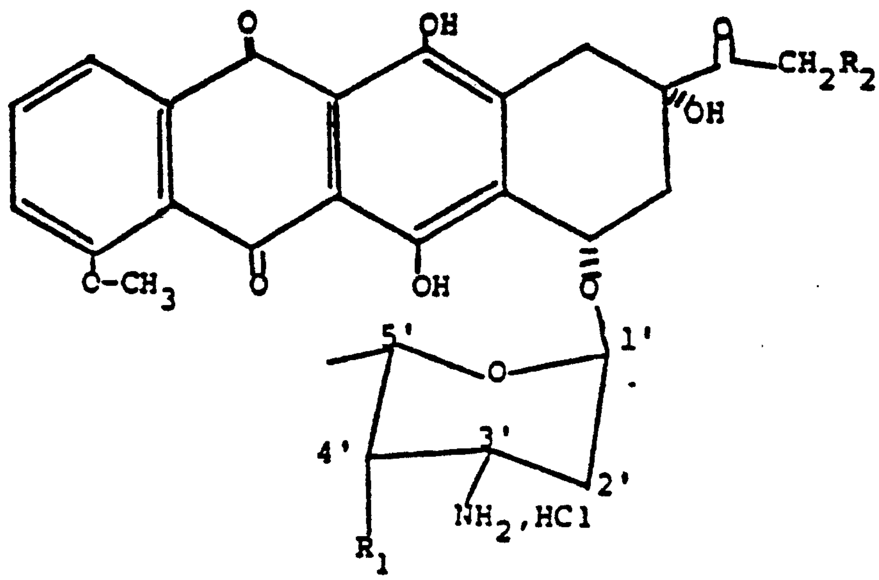

I

1 = $R_1$ = OH; $R_2$ = H (Daunorubicin)

2 = $R_1$ = $R_2$ = OH (Doxorubicin)

3 = $R_1$ = $R_2$ = H (EP-B-48 967)

Die Umkehr der Konfiguration des Zuckers in Position der 3'-und/oder 4'-Kohlenstoffatome (siehe untenstehende Formel II) ermöglicht ebenfalls die Herstellung pharmakologisch ausserordentlich interessanter Derivate.

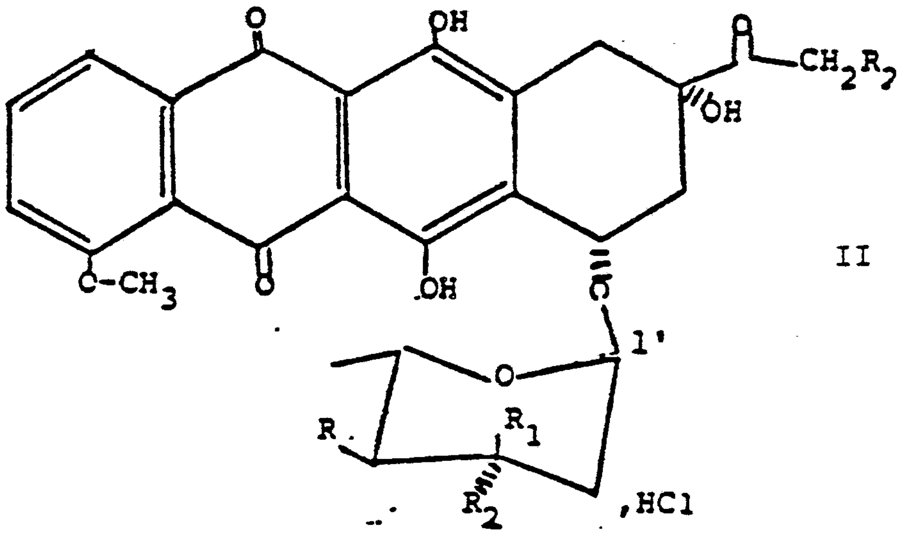

II

4 R = OH; $R_1$ = H; $R_2$ = $NH_2$

5 R = $R_2$ = H; $R_1$ = $NH_2$

Das Glykosid 4 weist eine starke, antitumorale Aktivität auf, während das desoxydierte Produkt 5 Gegenstand eines jüngst erteilten Patents ist [Carlo ErbaJpn. Kokai Tokkyo Koho Jp 57-142-981 (82-142-981), Farmitalia, BE-B-891 837].

Das Interesse an den beiden Aminodesoxyzuckern, welche Bestandteil der Anthracyclinzucker 3 und 5 bilden, steht deschalb ausser Zweifel. Bedauerlicherweise schränkt jedoch die Herstellungsweise dieser Zucker deren potentielle Möglichkeiten beträchtlich ein. So sind die verwendeten Verfahren abgeleitet von

-Anthracyclin selbst (wobei eine Desoxydierung an dem in 4'-Stellung befindlichen Kohlenstoffatom beispielsweise auf der Stufe des Produkts 1 lediglich für die ausschliessliche Herstellung des Produkts 3 Verwendung finden kann); oder von den

-Zuckern, welche man abwandlen kann. Es ist offensichtlich, dass letztgenannte Alternative wesentlich interessanter und auch von universellerer Bedeutung ist. Einzig der vorgeschlagene Herstellungsweg ist in wirtschaftlicher Hinsicht nicht tragbar, da er sehr kompliziert ist, viele Schritte umfasst und eine Reihe anderer Probleme bereitet, insbesondere die Bildung von halogenierten Zuckern, schwierig zu synthetisierenden und sehr häufig instabilen Produkten.

Folglich besteht die Aufgabe der vorliegenden Erfindung darin, eine Zuckersynthese vorzusehen, welche leicht, einfach und wirtschaftlich in der Weise vonstatten geht, dass zahlreiche Glykoside synthetisierbar sind.

Die vorliegende Erfindung hat ferner eine neue Familie von Zuckern, insbesondere 3-Amino-3,4,6-tridesoxyglykale der nachstehenden allgemeinen Formeln 14 oder 14

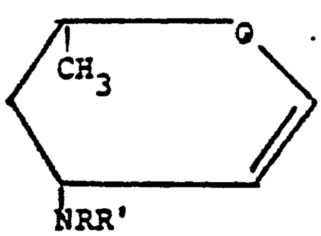

14

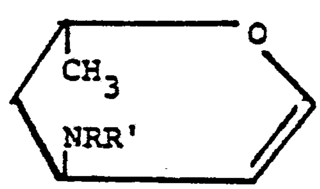

15

zum Gegenstand, in denen R oder R', welche gleich oder verschieden sein können, ein Wasserstoffatom oder eine gegebenenfalls substituierte Alkyl-bzw. Acylgruppe bedeuten oder R und R' zusammen mit dem Stickstoffatom die

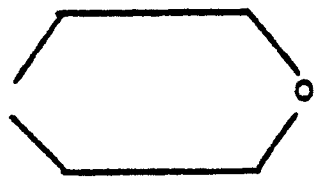

Morpholingruppe bilden.

Jüngste Veröffentlichungen haben in der Tat gezeigt, dass der Ersatz der Aminogruppe ($NH_2$) in 3'-Stellung am Daunorubicin durch eine Morpholingruppe

( 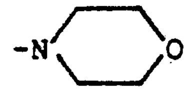 )

eine Erhöhung der antitumoralen Aktivität (in vivo) bewirkt (vergl. insbes. die Arbeiten von C.W. MOSHER et al, J. Med. Chem. 25, 18, 1982).

Gemäss einer besonders vorteilhaften Ausgestaltung der Erfindung bedeuten R und/oder R' die $COCF_3$-Gruppe.

Ein weiterer Gegenstand der vorliegenden Erfindung ist ein Verfahren zur Herstellung der erfindungsgemässen 3-Amino-3,4,6-tridesoxyglykale, welches dadurch gekennzeichnet its, dass man in erster Stufe (4)-Methylthiocarbonate der nachstehenden, allgemeinen Formeln 12 und 13

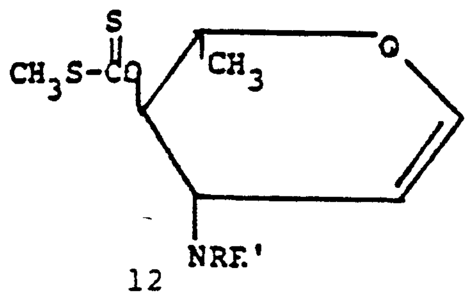

12

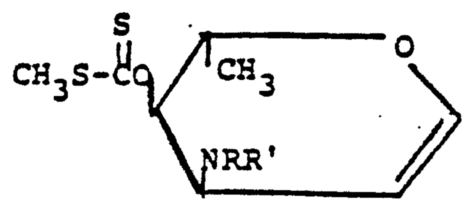

13

wobei R und R' dieselbe Bedeutung wie obenstehend definiert besitzen, mittels Reaktion von Glykalen der nachstehenden Formeln 10 und 11

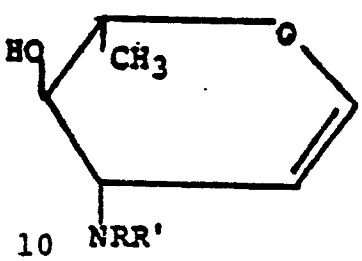

10

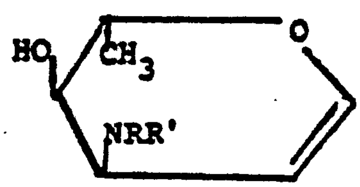

11

in denen R und R' die vorstehende Bedeutung haben, mit einem Gemisch aus Natriumhydrid und Schwefelkohlenstoff und nachfolgender Reaktion mit Methyljodid herstellt, und dass man in zweiter Stufe die Methylthiocarbonatgruppen mittels Tributylzinnhydrid in Gegenwart von Azobis-isobutyrontril einer reduzierenden Eliminierung unterzieht.

Die Glykale der Formel 10 und 11 sind bekannt. Ihre Herstellung wurde von J. BOIVIN, M. PAIS und C. MONNERET, Carbohydr. Res. 79, 193 (1980) beschrieben.

Die vorliegende Erfindung hat auch noch eine andere Verfahrensvariante zur Herstellung der erfindungsgemässen 3-Amino-3,4,6-triedesoxyglykalen zum Gegenstand, welche dadurch gekennzeichnet ist, dass man in erster Stufe Ester der nachstehenden allgemeinen Formeln 18 und 19

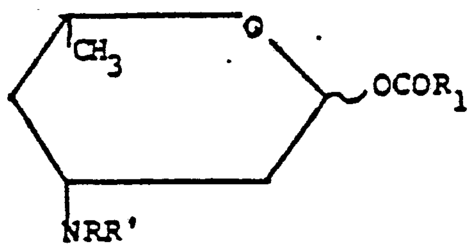

18

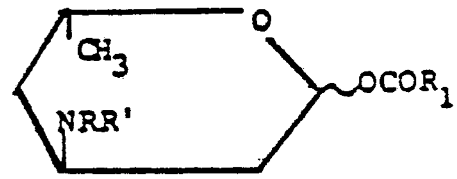

19

(wobei R und R′ dieselbe Bedeutung wie obenstehend definiert aufweisen und $R_1$ ein Wasserstoffatom oder eine gegebenenfalls substituierte Alkyl-bzw. Arylgruppe bedeutet) mittels Reaktion unter Einwirkung eines Säureanhydrids oder Säurehalogenids mit Aminozuckern der nachstehenden allgemeinen Formeln 16 und 17:

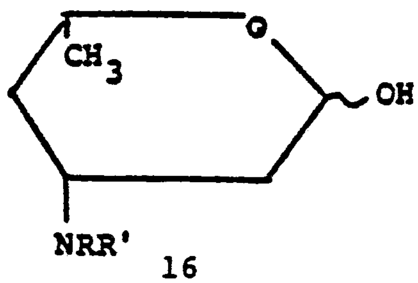

16

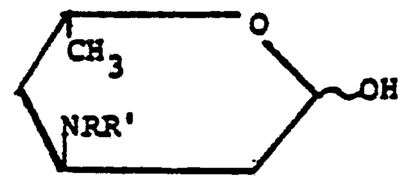

17

in denen R und R′ dieselbe Bedeutung wie oben aufweisen, in Anwesenheit einer Base herstellt, und dass man in zweiter Stufe die Glykale der Formeln 14 und 15 herstellt, welche man durch Erhitzen von Estern der Formeln 18 bzw. 19 gewinnt.

Gemäss einer bevorzugten Ausführungsform erhitzt man die Ester der Formeln 18 und 19 in Anwesenheit eines Adsorptionsmittels, wie z.B. Siliziumdioxyd ($SiO_2$). Die Aminozucker gemäss den Formeln 16 und 17 sind bekannt. Ihre Herstellung wurde von H.H. BAER und H.R. HANNA, Can. J. Chem. 58. 1751 (1980) beschrieben.

Ein weiterer Gegenstand der vorliegenden Erfindung sind mittels der erfindungsgemässen Glykale erhältliche Anthracycline sowie die Verwendung dieser Anthracycline allein oder in Mischung mit anderen Wirkstoffen bzw. Arzneimetteln.

Die Erfindung umfasst auch noch über den vorstehend geschilderten Rahmen hinausgehende Ausführungsformen, welche aus der nachstehenden Beschreibung hervorgehen.

Die vorliegende Erfindung zielt auf neue 3-Amino-3,4,6-tridesoxyglykale, Verfahren zu ihrer Herstellung, mit Hilfe von Glykalen durch Glykosidierung von Anthracyclinonen erhältliche Anthracycline, Arzneimittel mit einem Gehalt an Anthracyclinen wie auch geeignete Mittel zur Herstellung und Verwendung dieser Produkte ab.

Die Erfindung wird anhand der nachfolgenden, ergänzenden Beschriebung leichter verständlich gemacht. Sie erstreckt sich auf Beispiele für die erfindungsgemäss entwickelten Verfahren und schliesst auch pharmakologische Versuchsreihen mit ein.

Selbstverständlich dienen die durchgeführten und berücksichtigten Beispiele einzig zur Erläuterung des Erfindungsgegenstands, wobei keines der Beispiele irgendeine Beschränkung darstellen soll.

## BEISPIEL 1

Herstellung von 2,3,6-Tridesoxy-3-trifluoracetamido-4-methylthiocarbonate-L-arabino-1-hexenit (Verb. 12, R = H, R′ = $COCF_3$).

Man fügt zu einer Lösung von 1,5 g der Verb. 10 (6,6 mmol) (R = H, R′ = $COCF_3$) in 200 ml wasserfreiem Tetrahydrofuran 400 mg 80%iges Natriumhydrid (12 mmol) und hält das Gemisch 2 Std. unter Rühren in einer Argonatmosphäre bei einer Temperatur von 0°C. Danach setzt man 0,75 ml Schwefelkohlenstoff (12 mmol) und dann 0.75 ml Methyljodid (12,1 mmol) hinzu. Nach einstündigem

Rühren bei 0°C verdünnt man das Reaktionsgemisch mit Wasser, wonach man mit Aether extrahiert. Auf diese Wesie gewinnt man 1.7 g Rohsubstanz, welche über Kieselgur filtriert wird. Die Eluierung mittels eines Gemischs von Hexan und Dichlormethan im Verhältnis 2:1 ergibt 0,8 g (40% Ausbeute) der Verb. 12 - (R = H, R' = $COCF_3$) in reiner Kristallform: Schmelzpunkt = 145°C nach Umkristallisieren aus Hexan; $(\alpha)_D^{20}$ -170° (c-1, Chloroform);

IR: $\nu_{max}$ Nujol 3260 (NH), 1690 und 1655 $cm^{-1}$ ($NCOCF_3$); NMR: 270 MHz, $CDCl_3$): 6,55 (1H, NH, m); 6,40 (1H, dd, J = 5,5, J' = 1,8, H - 1); 5,89 (1H, t, J = J' = 9, H - 4); 4,88 (1H, m, J = J' = 9, J'' = 2, J''' = 1,8, H - 3); 4,68 (1H, dd, J = 5,5, J' = 1,8, H - 2); 4,18 (qd, J = 6,5, J' = 9, H - 5), 2,57 (3H, s, $SCH_3$); 1,32 (3H, d, J = 6,5, $CH_3$ -6).

## BEISPIEL 2

Herstellung von 2,3,4.6-Tetradesoxy-3-trifluoracetamid-L-threo-1-hexenit (Verb. 14, R = H, R' = $COCF_3$)

Man fügt zu einer Lösung von 0,7 g Verb. 12 (R = H, R' = $COCF_3$; 2 mmol) in 100 ml wasserfreiem Toluol 50 mg Azobisisobutyronitril unter Argonatmosphäre, wonach 1 ml Tributylzinnhydrid zugegeben wird. Das Reaktionsmedium wird über Nacht under Rückfluss erhitzt und danach bis zur Trockne eingedampft. Man suspendiert den Rückstand in Dichlormethan und fügt 1 g Kieselgur als Adsorptionsmittel hinzu. Die Entfernung eines Ueberschusses an Tributylzinnhydrid geschieht durch Filtrieren über 3,5 g Kieselgur in den ersten Fraktionen mit Hexan als Eluiermittel. In den folgenden Fraktionen werden 440 mg der Verb. 14 mit dem Eluiermittel Hexan-Dichlormethan im Verhältnis 2:1 isoliert (in Verb. 14 bedeuten R = H, R' = $COCF_3$). Die Ausbeute beträgt 95%. Eine Probe wurde für die Analyse in Hexan umkristallisiert; Schmelzpunkt = 101 - 102°C; $[\alpha]_D^{20}$ -29° (c-1, Chloroform);

IR:$\nu_{max}$ Nujol: 3330 (NH); 1690n und 1640 $cm^{-1}$ ($NHCOCF_3$); NMR: (270 MHz, $CDCl_3$): 6,42 (1H, d, J = 6, H-1); 6,15 (massiv, NH); 4,67 (1H, m, J = 10, J' = 9, J'' = 5, J''' = 2, H-3); 4,55 (1H, dd, J = 6, J' = 2, H-2); 4,08 (qd, J = 6,5, J' = 10, J'' = 1, H-5); 2,32 (m, J = 14, J' = 5, J'' = 1, H-4e); 1,48 (1H, m, J = 14, J' = J'' = 10, H-4a); 1,30 (3H, d, J = 6,5, $CH_3$-6).

## BEISPIEL 3

Herstellung von 2,3,6-Tridesoxy-3-trifluoracetamido-4-methylthiocarbonat-1-ribo-1-hexenit (Verb. 13, R = H, R' = $COCF_3$).

Man erhält die Verbindung 13 (R = H, R' = $COCF_3$) aus der Verbindung 11 (R = H, R' = $COCF_3$) auf die gleiche Weise wie die Verbindung 12 (R = H, R' = $COCF_3$) aus dem Produkt 10 (R = H, R' = $COCF_3$) Schmelzpunkt 92°C ($CH_2Cl_2$); $[\alpha]_D^{20}$ -73° (c-1, Chloroform);

IR: vergl. 12

NMR: (270 MHz, $CDCl_3$): 6,58 (1H, d, J = 5,5, J' ≃ 1, H-1); 6,56 (1H, m, J = 10, NH); 5,86 (1H, dd, J = 8, J' = 5, H-4); 4.92 (1H, m, J = 10, J' = J'' = 5, H-3); 4,73 (1H, dd, J = 5,5, J' = 5, H-2); 4,25 (1H, qd, J = 6,5, J' = 8, H-5); 2,57 (3H, s, $SCH_3$); 1,35 (3H, d, J = 6,5, $CH_3$-6).

## BEISPIEL 4

Herstellung von 2,3,4,6-Tetradesoxy-3-trifluoracetamido-L-erythro-1-hexenit (Verb. 15, R = H, R' = $COCF_3$).

Man erhält die Verbindung aus dem Produkt 13 (R = H, R' = $COCF_3$) in entsprechender Weise wie das Produkt 14 (R = H, R' = $COCF_3$) aus der Verbindung 12 (R = H, R' = $COCF_3$); Schmelzpunkt = 110° - 112°C (Hexan); $[\alpha]_D^{20}$ -76° (c-1, Chloroform);

IR: $\nu_{max}$ Nujol: vergl.14;

NMR: (270 MHz, $CDCl_3$): 6,49 (1H, d, J = 5,5, H-1); 6,46 (1H, massiv, NH); 4,75 (1H, m, J = J' = 5, J'' = 1, H-2); 4,33 (1H, m, H-3); 3,85 (1H, m, J = 6,5, J' = 10, J'' -2,5, H-5); 1,95 (2H, m, J = 14. J' = 2,5, J'' = 2,5, H-4e); 1,78 (2H, m, H-4a); 1,33 (3H, d, $CH_3$-6).

## BEISPIEL 5

Herstellung von 0-1-Acetyl-2,3,4,6-tetradesoxy-3-trifluoracetamido-L-threo-hexose (Verb. 18, R = H, R' = $COCF_3$, $R_1$ = $CH_3$).

Man lässt eine Lösung von 2,2 g 2,3,4,6-Tetradesoxy-3-trifluoracetamido- L -threo-hexose (Verb. 16, R = H, R' = $COCF_3$), (0.01 mol) in 80 ml Pyridin und 30 ml Essigsäurenhydrid über Nacht unter Rühren bei Raumtemperatur stehen. Die Methylenchloridextraktion nach konventioneller Methode ergibt 1,8 g Acetat (Verb. 18, 70%, α- L -und β- L -Enantiomere);
Schmelzpunkt/Gemisch 130 - 155°C
SM: m/z = 269 (M, nicht vorhanden); 209 (54%), 194 (20%); 180 (43%); (140 (100%).
IR: ($CH_2Cl_2$): 3440, 3060, 2995, 1750, 1730, 1560, 1450, 1430, 1380, 1370, 1330, 1320, 1280, 1230, 1175, 1150, 1115, 1060, 1040, 1020 $cm^{-1}$.
NMR ($CDCl_3$) α- L -Enantiomer: 6,62 ppm (NH); 6,22 (L $_{1/2}$ = 6 Hz, H-1); 2,10 (Singulett, $COCH_3$); 1,22 (Dublett, J = 6 Hz, $CH_3$).
β- L -Enantiomer: Schmelzpunkt = 162 - 163°C.
NMR ($CDCl_3$): 6,27 (NH); 5,67 (Dublett v. Dublett), J = 10 Hz; J = 2,5 Hz, H-1); 2,08 (Singulett, $COCH_3$); 1,27 (Dublett, J = 6 Hz, $CH_3$).

## BEISPIEL 6

Herstellung von 2,3,4,6-Tetradesoxy-3-trifluoracetamido-L-threo-1-hexenit (Verb. 14, R = H, R' = $COCF_3$)

Man bringt eine Lösung von 600 mg Acetat (Verb. 18, 2,23 mmol) in 100 ml Toluol in Anwesenheit von 10 g Kieselgel in einem mit einer sogenannten Dean-Stark-Vorrichtung ausgerüsteten Kolben zum Kochen. Nach einer Stunde filtriert man das Kieselgel ab und wäscht mit Aethylacetat nach. Die organische Lösung wird erst mit einer Hydrogencarbonatlösung und dann mit Wasser gewaschen. Nach dem Trocknen und Abdampfen der Lösungsmittel reinigt man die erhaltene Rohsubstanz durch schnelles Hindurchlaufenlassen über eine Kieselgelsäule. Man erhält so 210 mg (45%) Glykal der Formel 14 (R = H, R' = $COCF_3$), dessen Konstanten bereits unter Beispiel 2 angegeben sind.

## HERSTELLUNG DER ANTHRACYCLINE

Die Verknüpfung der Glykale der Formel 14 (R = H, R' = $COCF_3$) bzw. 15 (R = H, R' = $COCF_3$) mit Anthracyclinonen führt zu Anthracyclin-Verbindungen mit bemerkenswerten antitumoralen Wirkungen. Dieser Herstellungsweg umfasst generell 2 Stufen:

a) die Glykosidierung

b) die Entfernung der Schutzgruppe(n) für den Fall, dass R und R' in den Glykalen gemäss den Formeln 14 und 15 jeweils Wasserstoffatome darstellen und wenigstens ein Wasserstoffatom zur Durchführung der Reaktion geschützt werden muss.

Beispielsweise reagiert das Daunomycinon (Verb. 20) mit dem Glykal (Verb. 14, R = H, R' = $COCF_3$) in verschiedenen wasserfreien Lösungsmitteln (z.B.: $CH_2Cl_2$, Benzol, und dgl.) in Anwesenheit von Säure (z.B. Paratoluolsulfonsäure) bei Raumtemperatur.

Daneben erhält man die Verbindung 21, deren Herstellung bereits beschrieben ist, sowie die neuen Stoffe 22a und 23a.

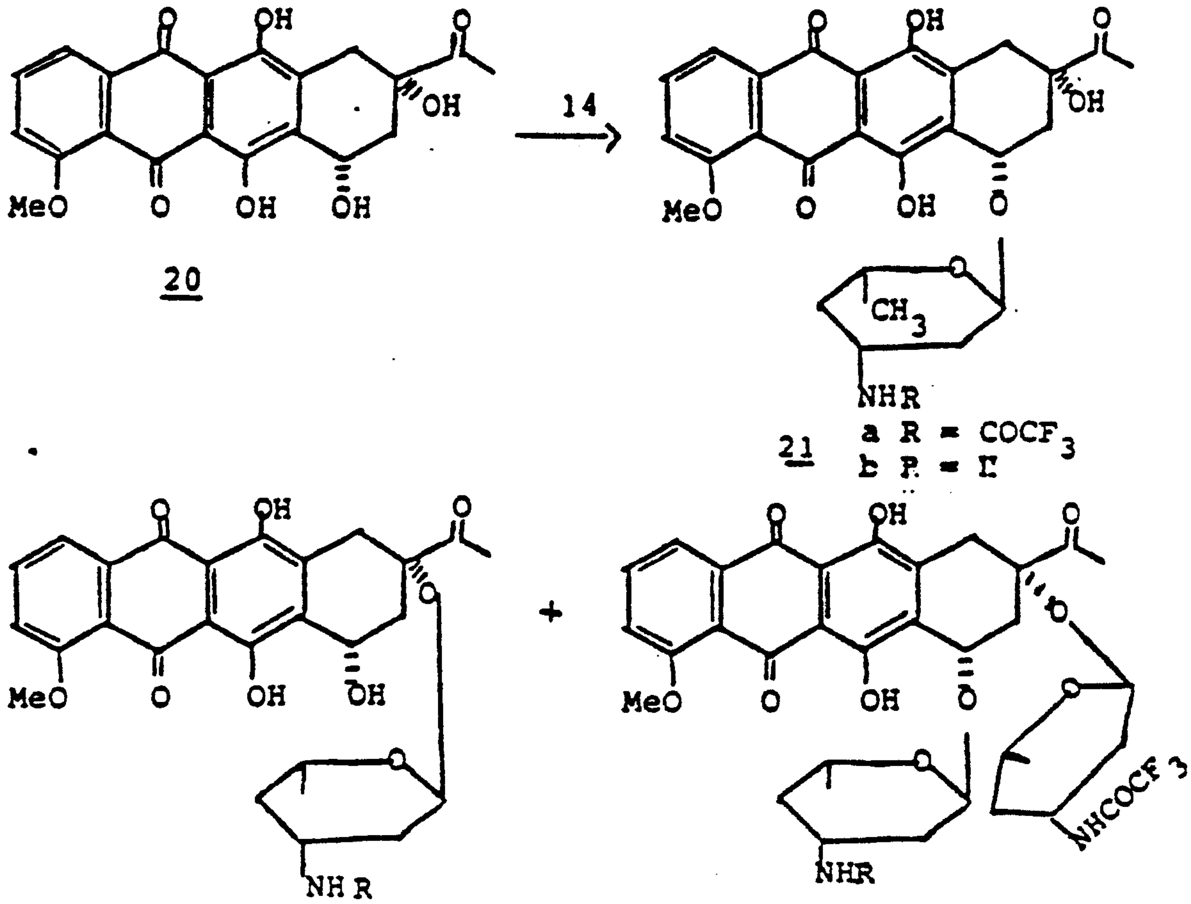


20

21 a R = $COCF_3$
b R = H

22 a R = $COCF_3$
b R = H

23 a R = $COCF_3$
b R = H

BEISPIEL 7

Glykosidierungsreaktion das Daunomycinons 20 mittels Glykal (Verb. 14, R = H, R' = $COCF_3$)..

(Glykosidierungsschritt)

Man gibt zu einer Lösung von 71 mg Daunomycinon 20 (0,178 mmol) und 149 mg Glykal (Verb. 14, R = H, R' = $COCF_3$) (0,71 mmol) in 15 ml über $P_2O_5$ destilliertem $CH_2Cl_2$ die katalytische Menge von 2,3 mg Paratoluolsulfonsäureanhydrid (0.013 mmol).

Man giesst das Reaktionsgemisch nach 4-stündigem Rühren bei Raumtemperatur zu einer Natriumhydrogencarbonatlösung und extrahiert mit Methylenchlorid in konventioneller Weise. Die Auftrennung auf Kieselgelplatten ergibt die drei gebildeten Glykoside (Laufmittel: Hexan/Aethylacetat 50:50):

-Das 7-0-(2', 3', 4', 6'-Tetradesoxy-3α-trifluoracetamido-L -threo-hexopyranosyl)-daunmycinon (Verb. 21a; 40%; $R_f$ = 0,26 mit Hexan/Aethylacetat 50:50);

-das 9-0-(2',3',4',6'-Tetradesoxy-3α-trifluoracetamido- L -threo-hexopyranosyl)-daunomycinon (Verb. 22a; 35%; $R_f$ = 0,28 mit Hexan/Aethylacetat 50:50);

-das 7,9-Di-0-(2', 3', 4', 6'-tetradesoxy-3α-trifluoracetamido-1-threo-hexopyranosyl)-daunomycinon (Verb. 23a; 23% $R_f$ = 0,34 mit Hexan/Aethylacetat 50:50).

Produkt 21a: Ausbeute 40%

Schmelzpunkt = 140 - 145°C (Zersetzg.)

$\alpha_D$ = 458° (C = 0,23; $CHCl_3$)

IR (KBr): 3500, 3330, 2940, 1705 (Weit), 1610, 1570, 1400, 1350, 1280, 1190, 1120, 1030, 980 $cm^{-1}$.

NMR ($CDCl_3$): 1,3 (Dublett, 3H); 2,4 (Singulett, 3H), 4,4 (Singulett, 1H), 5,22 (Multiplett, $L_{1/2}$ = 7 Hz, 1H); 5,56 (Multiplett, $L_{1/2}$ = 6,5 Hz, 1H); 6.10 (weit, 1H); 3,1 (Singulett, 1H); 13,85 (Singulett, 1H).

$R_f$ = 0,7 ($CH_2Cl_2$-MeOH, 96-4)

$R_f$ = 0,26 (Hexan/Aethylacetat, 50:50).

Produkt 22a: Ausbeute 35%

Schmelzpunkt = 164 - 169°C (Zersetzg.)

$\alpha_D$ = +290° (C = 0,18: $CHCl_3$)

IR (KBr): 3320 (weit), 2980, 2940, 1720, 1710, 1610, 1580, 1550, 1450, 1415, 1380, 1350, 1290, 1250, 1210, 1140, 1070, 1040, 1010, 990, 920 $cm^{-1}$.

NMR ($CDCl_3$): 2,37 (Singulett, 3H); 4,03 (Singulett, 3H); 4,98 (Multiplett, $L_{1/2}$ = 8 Hz, 1H); 5,42 (Multiplett, $L_{1/2}$ = 8 Hz, 1H); 13,27 (Singulett, 1H); 13,79 (Singulett, 1H).

$R_f$ = 0.66 ($CH_2Cl_2$-MeOH, 96-4).

$R_f$ = 0,28 (Hexan/Aethylacetat, 50:50).

Produkt 23a: Ausbeute 23%

Schmelzpunkt = 227 - 228°C (Zersetzg.)

$\alpha_D$ = 0,5° (C = 0,27, $CHCl_3$).

IR (KBr): 3440, 3320, 2980, 2920, 1725, 1710, 1605, 1580, 1550, 1410, 1350, 1295, 1270, 1230, 1200, 1150, 1100, 1080, 1060, 1030, 1000, 980, 915 $cm^{-1}$ kl.

NMR ($CDCl_3$): 0,53 ppm (d, J = 6 Hz, 3H), 1,32 (d, J = 6 Hz, 3H); 2,31 (s, 3H); 4,03 (s, 3H); 4,97 (Multiplett, $L_{1/2}$ = 8 Hz, 2H); 5,47 (Multiplett, $L_{1/2}$ = 8 Hz, 2H); 6,20 (weit, NH, 2H); 13,23 (Singulett, 1H); 13,79 (Singulett, 1H).

$R_f$ = 0,58 ($CH_2Cl_2$/MeOH, 96-4).

$R_f$ = 0,34 (Hexan/Aethylacetat, 50:50).

## BEISPIEL 8

Herstellung von Di-0-7,9-(3'-amino-2',3',4',6'-tetradesoxy-α-1-threo-hexopyranosyl)-daunomycinon•HCl (Verb. 23b)

(Stufe zur Entfernung der Schutzgruppen)

Die Entfernung der Schutzgruppen ($COCF_3$) geschieht bei den Verbindungen der Formeln 21a und 23a in alkalisch-wässrigem Medium.

Man fügt zu einer Lösung von 20 mg Glykosid der Formel 23a (0,024 mmol) in 8 ml Tetrahydrofuran 8 ml 0,1N Natriumcarbonatlösung bei 0°C und unter Stickstoffatomosphäre hinzu. Nach 14-stündiger Reaktionszeit bei 0°C wird der pH-Wert auf 4 - 4,5 mittels 0,1N Salzsäure eingestellt. Danach wird die wässrige Phase mehrere Male mit Methylenchlorid gewaschen, mit Natriumhydrogencarbonatlösung alkalisch gemacht und in konventioneller Weise mit Methylenchlorid extrahiert.

Das so erhältliche 7,9-Di-0-(3'-amino-(2', 3', 4', 6'-tetradesoxy-α- L -threo-hexopyranosyl)-daunomycinon (Verb. 23b ) wird in 2 ml wasserfreiem $CH_2Cl_2$ gelöst und mit einem Aequivalent HCl in 0,05N methanolischer Lösung in Reaktion gebracht. Das gebildete Hydrochlorid wird mit Aether ausgefällt (Schmelzpunkt = 173 - 174°C; Zersetzg.).

## BEISPIEL 9

Herstellung von 9-0-(3'-Amino-(2',3',4',6'-tetradesoxy-α-L-threo-hexopyranosyl)-daunomycinon•HCl (Verb. 22b).

Man erhält das Chlorhydrat (Schmelzpunkt = 175 - 177°C, Zersetzg.) in derselben Weise wie in Beispiel 8 beschrieben.

NMR ($CDCl_3$): 2,3 ppm (Singulett, 3H); 4,0 (Singulett, 3H); 4,90 (Multiplett, 1H); 5,32 (Multiplett, 1H).

## BEISPIEL 10

Herstellung von 7-0-(3'-Amino-(2',3',4',6'-tetradesoxy-α-L-threo-hexopyranosyl)-daunomycinon bzw. (4'-Desoxy-daunomycin) = Verb. 21b.

Dieser Stoff wird ebenso wie in Beispiel 8 erläutert, synthetisiert und mittels Kieselgel-Chromatographie auf Platten gereinigt (Laufmittel $CHCl_3$-MeOH-$NH_4OH$; 180-20-1).

NMR ($CDCl_3$): 2,37 (Singulett, 3H), 4,0 (Singulett, 3H); 5,25 (Multiplett, $L_{1/2}$ = 7 Hz, 1H); 5,50 (Multiplett $L_{1/2}$ = 8 Hz, 1H).

## BEISPIEL 11

Herstellung von 7-0-(3'-Morpholino-(2',3',4',6'-tetradesoxy-α- L -threo-hexopyranosyl)daunomycinon•HCl (bzw. 3'-Desamino-4'-desoxy-3'-morpholino-aunomycin•HCl) = Verb. 24.

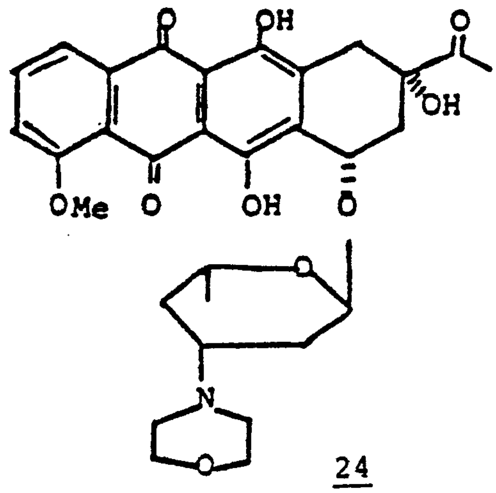

24

Man lässt eine Lösung von 38 mg 4'-Desoxy-daunomycin der Formel 21b (0,074 mmol) in 2 ml DMF mit 411 mg Di-(2-jodäthyl)äther (1,26 mmol) sowie 22 mg Triäthylamin (0,22 mmol) bei Raumtemperatur 36 Stunden lang reagieren. Durch Extraktion und Reinigung mittels Kieselgelplatten (Laufmittel $CHCl_3$-MeOH-$NH_4OH$) erhält man die Verbindung 24 (60%). Man behandelt die Lösung letztgenannter Verbindung in 2 ml $CH_2Cl_2$ mit einm Aequivalent HCl in 0,05N methanolischer Lösung. Das gebildete Hydrochlorid wird mit Aether ausgefällt.

Schmelzpunkt = 145 - 150°C (Zersetzg.).

NMR ($CDCl_3$) 24: 1,27 (Dublett, 3H, $CH_3$); 2,40 (Singulett, 3H, $CH_3$); 2,44 (Multiplett, $CH_2$ x 2); 3,61 (Multiplett, $CH_2$ x 2); 4,78 (Multiplett, 1H); 5,25 (Multiplett, 1H); 5,55 (Multiplett, 1H); 13,82 (Singulett, 1H).

## BEISPIEL 12

Reaktion zur Verknüpfung des Glykals der Formel 15.

Das nach dem Verfahren nach Jean-Claude FLORENT und Claude MONNERET (FR-B-84-03634) hergestellte Aglykon der Formel 25 reagiert mit dem Glykal der Formel 15 (R = H, R' = $COCF_3$) in verschiedenen wasserfreien Lösungsmitteln bei Raumtemperatur in Anwesenheit von p-Toluolsulfonsäure.

Man erhält drei neue Stoffe, nämlich die Anthracycline 26, 27und 28.

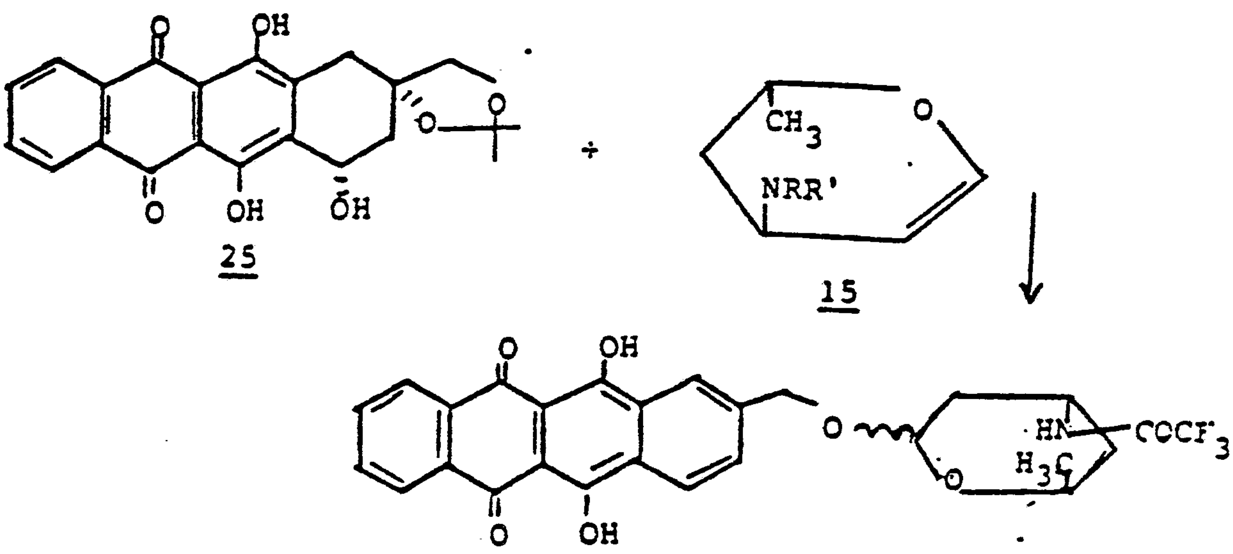

25

15

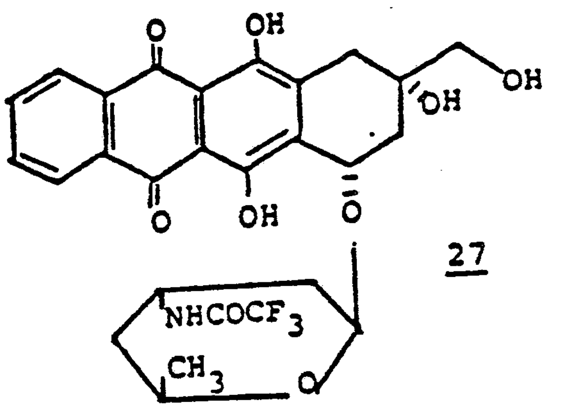

27

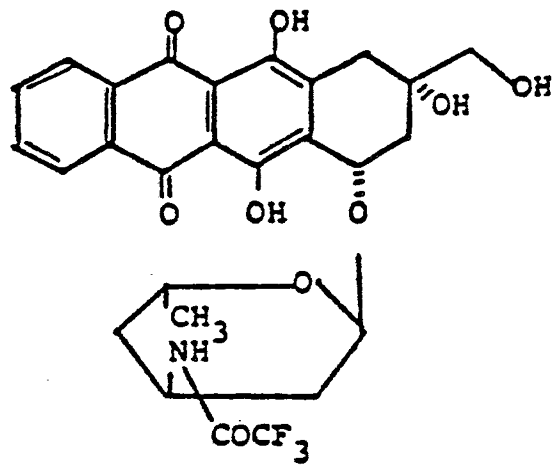

Man fügt 70 mg p-Toluolsulfonsäure (0,41 mmol) zu einer Lösung von 70 mg der Verbindung 25 (0,176 mmol) und 70 mg Glykal 15 (R = H, R' = $COCF_3$, 0,33 mmol) in 15 ml wasserfreiem Benzol. Nach 2-stündigem Rühren bei Raumtemperatur giesst man das Reaktionsgemisch in Natriumhydrogencarbonatlösung und extrahiert dann in konventioneller Weise mittels Dichlormethan. Dann isoliert man 150 mg Rohsubstanz nach dem Auswaschen mittels organischem Lösungsmittel und dessen Abdampfen unter verringertem Druck.

Mittels Kieselgelchromatographie (Laufmittel: reines Toluol) lässt sich die Rohsubstanz in drei Glykoside auftrennen:

-Das 13-0-(2',3',4',6'-Tetradesoxy-3-trifluoracetamido-α und β- L -erythro-hexopyranosyl)-6,11-dihydro-5,12-dihydroxy-2-hydroxymethyl-naphthacen-6,11-dion (25%, $R_f$ = 0,43, Toluol/Aceton 90:10) = Verb. 26.

-Das 1-0-(2',3',4',6'-Tetradesoxy-3-trifluoracetamido-β- L -erythro-hexopyranosyl)-1,2,3,4,6,11-hexhydro-1(S), 3(S), 5,12-tetrahydroxy-3-hydroxymethyl-naphthacen-6,11-dion (45%, $R_f$ = 1,15, Toluol/Aceton 90:10) = Verb. 27.

-Das 1-0-(2',3',4',6'-Tetradesoxy-3-trifluoracetamido-α- L -erythro-hexopyranosyl)-1,2,3,4,6,11-hexhydro-1(S)-,3(S), 5, 12-tetrahydroxy-3-hydroxymethyl-naphthacen-6,11-dion (25%, $R_f$ = 0,08, Toluol/Aceton 90:10) = Verb. 28.

I - Anthracyclin 26: 13-0-(2',3',4',6'-Tetradesoxy-3-trifluoracetamido-α und β- L -erythro-hexopyranosyl)-6,11-dihydro-5,12-dihydroxy-2-hydroxymethyl-naphthacen-6,11-dion

Schmelzpunkt des kristallinen Gemischs = 155 - 157°C (Toluol); $[\alpha]_D$ +17° (c 0,03, THF);
IR ($CHCl_3$): 3380, 2920, 1720, 1590 , 1540, 1510, 1460 (verbreitert), 1410, 1340, 1265, 1170, 1115, 1040 980 $cm^{-1}$;
NMR ($CDCl_3$): 15,65 (OH); 8,55 - 7,77 (Multiplett, 7H aromat. und NH): 5,17 (Singulett, erweitert, H-1' äqu.)

und 5,00 (Dublett, H-1' axial); 4,95 (Dublett) und 4,73 (Dublett) (Syst. AB, $CH_2$-13); 4,00 (Multiplett, 2H, H-5' und H-3'); 2,00 - 1,44 (Multiplett, $CH_2$-2' und 4'); 1,28 -1,15 (Multiplett, 6H, $CH_3$-6').
MS: ($DCI/NH_3$): m/z 547 (M + 18, 1%); 530 (M + 1, 10%); 321 (Aglukon + 1, 15%); 245 (Zucker + 18, 15%); 227 (Zucker, 25%); 210 (Zucker - 18, 100%).
$R_f$ = 0,64 ($CH_2Cl_2$/MeOH, 98:2).
$R_f$ = 0,25 (Hexan/Aetylacetat, 66:33).

II. Anthracyclin 27

Schmelzpunkt 182 - 183°C (Hexan); $[\alpha]^{20}_{D}$ +139°, c 0,02, THF)
IR ($CHCl_3$): 3400, 2920, 1720, 1625, 1590, 1530, 1405, 1370, 1170, 990 und 970 $cm^{-1}$.
NMR ($CDCl_3$): 13,53 und 13,26 (2 Singul., 2H phenol, OH); 8,33 (Multiplett, 2H, Ar); 7,78 (Multiplett, 2H, Ar); 5,45 (Multiplett, 1H $L_{1/2}$ = 7 Hz, H-7); 5,38 (Dublett, 1H, J = 12, J' = 3, H-1'); 4,40 - 4,15 (Multiplett, 2H, H-3' und OH); 4,00 (Multiplett, 1H, H-5'); 3,81 (Dublett, 1H, H-13); 3,45 Dublett, 1H, H-13); 3,32 (halb. Dublett, 1H, H-10); 2,78 (halb. Doublett, 1H, H-10); 2,12 - 1,35 (Multiplett, 6H, 3 $CH_2$); 1,23 (Dublett, 3H, J = 6,5 Hz, $CH_3$-6').
MS ($DCI/NH_3$): m/z 583 (M + 18, ≈ 1%); 565 (M+, Spuren); 454 (7,5%); 374 (Aglukon + 18, Spuren); 356 (Aglukon, 5%); 341 (Aglukon - 15, 1%); 338 (Aglukon - 18, Spuren); 245 (Zucker + 18, 90%); 227 (Zucker, 25%); 210 (Zukker - 18, 100%).
$R_f$ = 0,27 ($CH_2Cl_2$:MeOH, 98:2).
$R_f$ = 0,10 (Hexan/Aethylacetat, 66:33).

III. Anthracyclin 28

Schmelzpunkt 162° - 164°C (Aceton); $[\alpha]^{20}_{D}$ + 140° (c 0,02, THF)
IR ($CHCl_3$): vergl. 30
NMR ($CDCl_3$): 13,53 und 13,26 (2 Singul. 2H, phenol. OH); 8.34 (Multiplett, 2H Ar); 7.83 (Multiplett, 2H, Ar); 5,33 (Dublett, 1H, J = 3 Hz, H-1'); 5,07 (Multiplett, 1H, $L_{1/2}$ = 7 Hz, H-7); 4,44 - 4,22 (Multiplett, 2H, H-3' und OH); 4,06 (Multiplett, H-5'); 3,90 (Dublett, 1H, J = 10 Hz, H-13); 3,45 (Dublett, 1H, J = 10 Hz, H-13); 3,20 (halb. Dublett, 1H, J = 18, J' = 1,5 Hz, H-10); 2,64 (Dublett, 1H, J = 18, H-10); 2,12 - 1,35 (Multiplett, 6H, $CH_2$-2',4' und 2); 1,23 (Dublett, 1H, J = 6,5 Hz, $CH_3$-6').
MS ($DCI/NH_3$): m/z 583 (M + 18, 2%); 566 (M + 1, 1%); 454 (5%), 374 (Aglukon + 18, 16%); 356 (Aglukon, 35%); 339 (Aglukon - 18, 7%); 245 (Zucker + 18, 30%); 227 (Zucker, 15%); 210 (Zucker - 18, 100%).
$R_f$ = 0,20 ($CH_2Cl_2$:MeOH, 98:2).
$R_f$ = 0,05 (Hexan:Aethylacetat 66:33).

PHARMAKOLOGISCHER VERSUCHSBERICHT:

I. Wirkung auf leukämische Zellen L 1210

A. Methodik:

Der Test wird nach der Methode von Hamburger und Salmon, modifiziert wie folgt, durchgeführt:

Das üblicherweise verwendete Medium wird durch jenes von Mc Coy 5 A ersetzt. Die Anzahl der in die Flaschen verbrachten Zellen wird auf 5 x $10^2$ pro Flasche aufgrund des schnellen Wachstums der Leukämiezellen L 1210 verringert.

Die Zellen werden innerhalb 1 Stunde bei 37°C in Anwesenheit der Testsubstanz in verschiedenen Konzentrationen inkubiert. Sodann werden die Zellen 2mal unter Verwendung des Mc Coy 5 A-Mediums auf Agaroseplatten nach der Methode von Hamburger und Salmon gewaschen.

Des weiteren wird der Test parallel mit der fortgesetzten Inkubation mit verschiedenen Konzentrationen der untersuchten Substanz durchgeführt, wobei die Substanz vor der Kultivierung der Zellen in die Agarose eingetragen wird.

Man stellt die Flaschen in den Wärmeschrank bei 37°C, dessen Atmosphäre einen Gehalt an 5 bzw. 20% $CO_2$ und eine relative Luftfeuchte von 95% aufweist, und beläst sie dort 5 bis 7 Tage lang. Nach diesem Zeitraum zählt man alle Kolonien mit einem Durchmesser von mehr als 60 µm mit Hilfe eines Umkehrmikroskops aus.

B. Ergebnisse

Die in Tabellen I wiedergegebenen Ergebnisse sind in Prozenten anhand der aus den L 1210 hervorgegangenen Kolonien im Vergleich zu den nicht behandelten Kontrollen ausgedrückt. Der im Verlauf der wiederholten Tests ermittelte Variationskoeffizient beträgt weniger als 15%.

**ERGEBNISSE** **TABELLE I**

| Substanz | | $ED_{50}(IC_{50})$ (µg/ml) kontin. Exposition |
|---|---|---|
| Verbindung 22b In Form des Hydrochlorids | 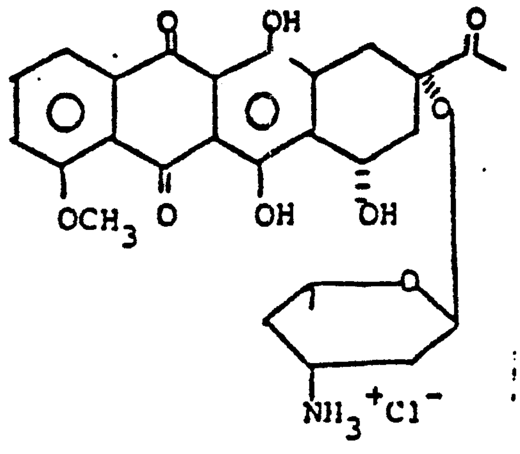 22b | 0,8 |
| Verbindung 23b in Form des Hydrochlorids | 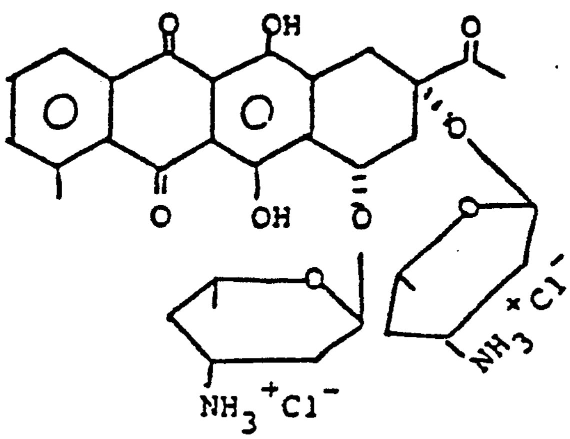 23b | 0,31 |
| Verbindung 24 in Form des Hydrochlorids | 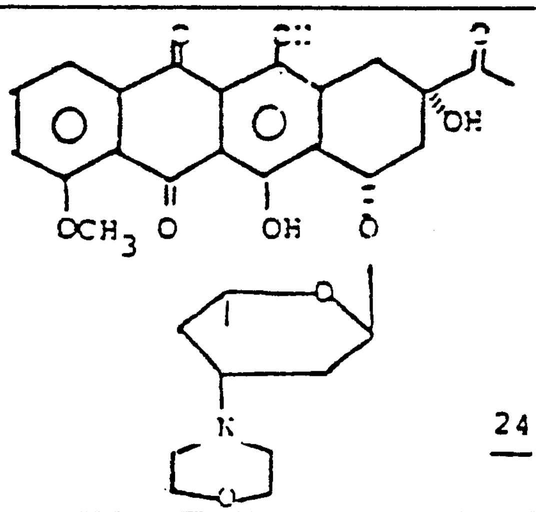 24 | $1,6 \times 10^{-3}$ |
| Adriamycin | | $2.10^{-2}$ |

Die $ED_{50}$($IC_{50}$)-Werte wurden anhand der Dosis/Wirkungskurven nach 1-stündiger kontinuierlicher Inkubation ermittelt.

II. Proliferationstest (Thymidinaufnahme)

A. Methodik:

Man inkubiert L 1210-Leukämiezellen während der exponentiellen Wachstumsphase (5 x $10^3$/ml in RPMI 1640) auf einer Mikrotitrationsplatte mit 96 Vertiefungen im Verlauf von 72 Stunden (37°C, 5% $CO_2$, relative Luftfeuchte 95%) mit unterschiedlichen Konzentrationen der jeweils ausgetesteten Substanzen. Die Kontrollen bestehen aus Zellen, welche in kühlem Kulturmedium exponiert worden waren. Für jede Konzentration werden 4 Vertiefungen vorbereitet. Nach Ablauf von 65 Stunden fügt man 50 µl 14C-Thymidin (1,5 µCi/ml) zur Markierung von zellulärer DNS hinzu. Nach 7-stündiger Inkubation werden die Zellen (mit dem Multimask cells harvester, Dynatec) gesammelt, sodann mit 5%iger Trichloressigsäure, - schliesslich mit Wasser und Methanol gewaschen und bei 50°C 2 Stunden lang getrocknet. Man bestimmt den Szintillationsindex mit Hilfe von 5 ml Szintillationsflüssigkeit (Rotiszint 11).

Die Ergebnisse sind in Form der Bezugswerte zwischen dem Szintillationsindex nach Inkubation mit den untersuchten Substanzen im Vergleich zu den Kontrollen ausgedrückt.

Der den wiederholten Experimenten zugrunde liegende Variationskoeffizient beträgt weniger als 15%.

B. Ergebnisse

Die $ED_{50}$($IC_{50}$)-Werte wurden anhand der Dosis/Wirkungskurven ermittelt.

**ERGEBNISSE** **TABELLE II** $ED_{50}(IC_{50})$ (µg/ml)

| Testsubstanz | | |
|---|---|---|
| Verbindung 22b in Form des Hydrochlorids | 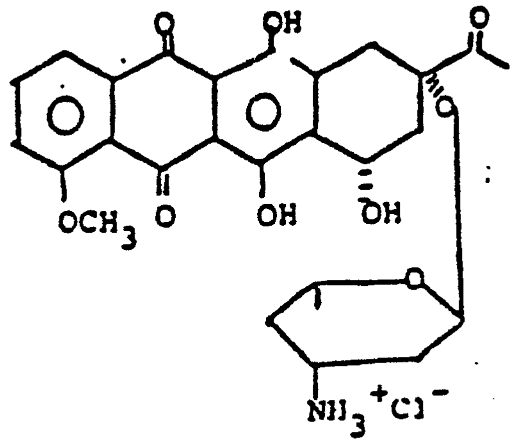 22b | 0,26 |
| Verbindung 23b in Form des Hydrochlorids | 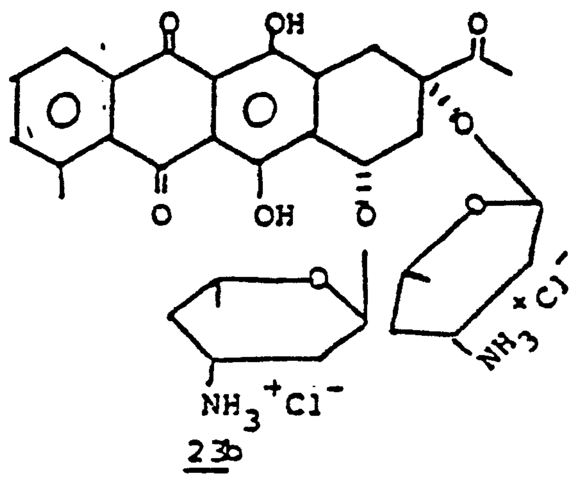 23b | 0,3 |
| Verbindung 24 in Form des Hydrochlorids | 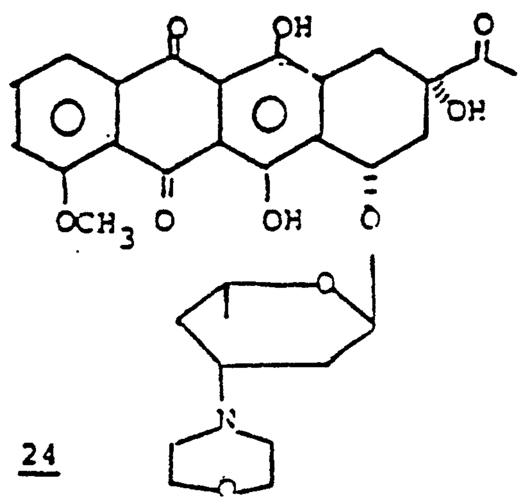 24 | $3.10^{-3}$ |
| Adriamycin | | $2,2.10^{-2}$ |

Die in Tabellen I und II aufgeführten Ergebnisse sind sehr vielversprechend. insbesondere die bei der Verbindung 24 festgestellten Resultate.

Die täglichen Humandosen liegen in der Grössenordnung von 1 bis 2 mg/kg.

Der vorstehend erläuterte Bereich der Erfindung erstreckt sich keineswegs auf die konkret beschriebenen Ausführungsbeispiele und Verwendungen; er umfasst vielmehr alle Abwandlungen, welche dem Fachmann auf diesem Gebiet gegeben sind, ohne über den Rahmen bzw. Umfang der vorliegenden Erfindung hinauszugehen.

**Ansprüche**

1. 3-Amino-3,4,6-tridesoxyglykale der nachstehenden allgemeinen Formeln 14 und 15:

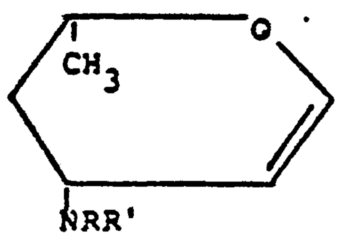

14

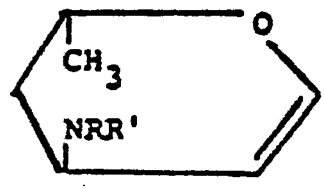

15

in denen R und R', welche gleich oder verschieden sein können, ein Wasserstoffatom oder eine gegebenenfalls substituierte Alkyl-oder Acylgruppe bedeuten, wobei R und R' zusammen auch eine Gruppe der Formel

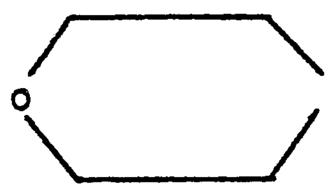

bedeuten können, welche mit dem Stickstoffatom die Morpholingruppe bildet.

2. 3-Amino-3,4,6-tridesoxyglykale nach Anspruch 1, dadurch gekennzeichnet, dass R und/oder R' die Gruppe $COCF_3$ bedeuten.

3. Verfahren zur Herstellung von 3-Amino-3,4,6-tridesoxyglykalen nach den Ansprüchen 1 und 2, dadurch gekennzeichnet, dass man in erster Stufe (4)-Methylthiocarbonate der nachstehenden allgemeinen Formeln 12 und 13:

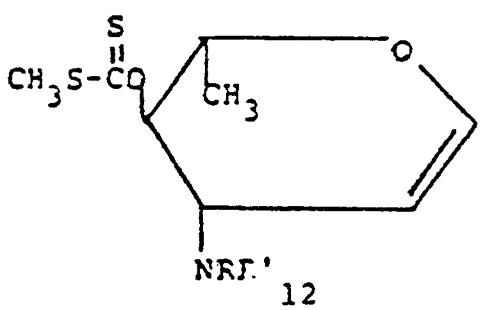

12

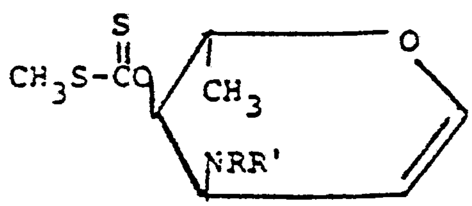

13

wobei R und R' dieselbe Bedeutung wie obenstehend definiert besitzen, mittels Reaktion von Glykalen der nachstehenden Formel 10 und 11:

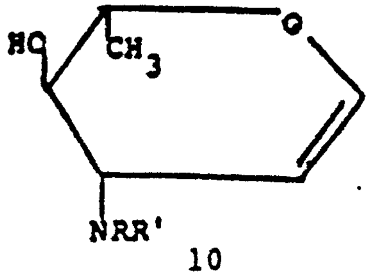

10

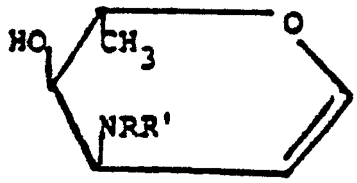

11

in denen R und R′ die vorstehende Bedeutung haben, mit einem Gemisch aus Natriumhydrid und Schwefelkohlenstoff und nachfolgender Reaktion mit Methyljodid herstellt, und dass man in zweiter Stufe die Methylthiocarbonatgruppen in Gegenwart von Azobis-isobutyronitril einer reduzierenden Eliminierung mittels Tributylzinnhydrid unterzieht.

4. Verfahren zur Herstellung von 3-Amino-3,4,6-tridesoxyglykalen nach den Ansprüchen 1 oder 2, dadurch gekennzeichnet, dass man in erster Stufe Ester der nachstehenden allgemeinen Formeln 18 und 19:

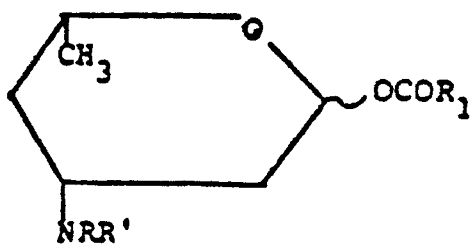

18

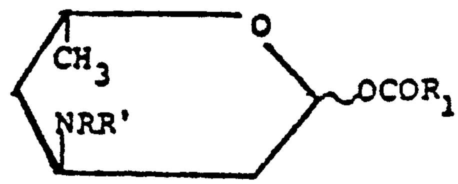

19

(wobei R und R′ dieselbe Bedeutung wie obenstehend definiert aufweisen und $R_1$ ein Wasserstoffatom oder eine gegebenenfalls substituierte Alkyl-bzw. Arylgruppe bedeutet) mittels Reaktion eines Säureanhydrids oder Säurehalogenids mit Aminozuckern der nachstehenden allgemeinen Formeln 16 und 17:

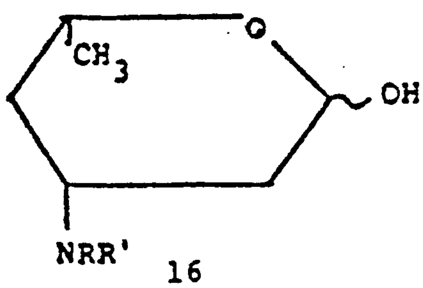

16

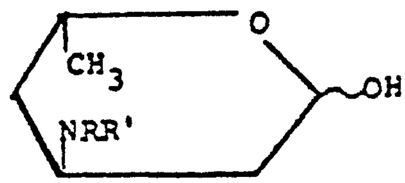

17

in denen R und R′ dieselbe Bedeutung wie obenstehend aufweisen, in Anwesenheit einer Base herstellt, und dass man in zweiter Stufe die Glykale der Formeln 14 und 15 herstellt, welche man durch Erhitzen von Estern der Formeln 18 bzw. 19 gewinnt.

5. Verfahren nach Anspruch 4, dadurch gekennzeichnet, dass man das Erhitzen der Ester gemäss Formeln 18 und 19 in Anwesenheit eines Adsorptionsmittels durchführt.

6. Anthracycline, dadurch gekennzeichnet, dass sie mittels der Glykale nach den Ansprüchen 1 oder 2 hergestellt wurden.

7. Anthracycline nach Anspruch 6, dadurch gekennzeichnet, dass sie den Formeln 22a und 22b:

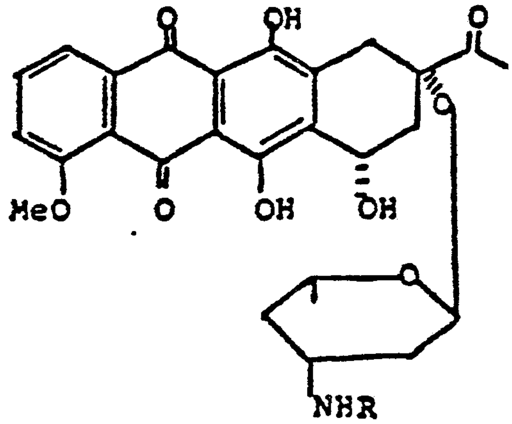

22a R = $COCF_3$

b R = H

entsprechen.

8. Anthracycline nach Anspruch 6, dadurch gekennzeichnet, dass sie den Formeln 23a und 23b:

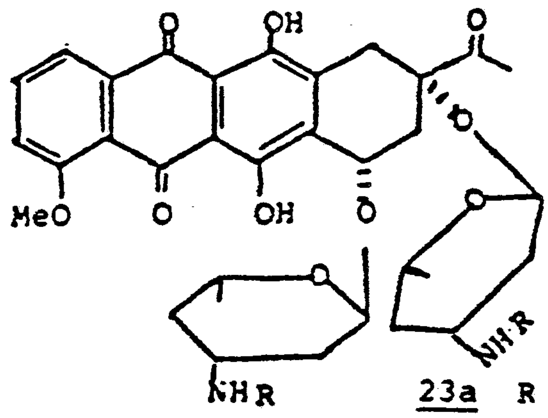

23a R = $COCF_3$

b R = H.

entsprechen.

9. Verwendung von Anthracyclinen nach einem der Ansprüche 6 bis 8 als Arzneimittel.